# EUROPÄISCHE PATENTSCHRIFT

(11) **EP 3 628 365 B1**
(45) Veröffentlichungstag und Bekanntmachung des Hinweises auf die Patenterteilung: **16.08.2023**
(21) Anmeldenummer: 19197947.5
(22) Anmeldetag: 18.09.2019
(51) Int. Cl.: A61M 39/10

(54) **VERBINDUNGSVORRICHTUNG FÜR DIE WUNDVERSORGUNG UND WUNDVERSORGUNGSKIT**
CONNECTION DEVICE FOR WOUND CARE AND WOUND CARE KIT
DISPOSITIF DE RACCORDEMENT POUR LE SOIN DE PLAIES ET ENSEMBLE DE SOIN DE PLAIE

(30) Priorität: 28.09.2018 DE 202018105593 U
(43) Veröffentlichungstag der Anmeldung: 01.04.2020
(73) Patentinhaber: Lohmann & Rauscher GmbH, 2525 Schönau/Triesting (AT)
(72) Erfinder: Herzele, Arno, 2353 Guntramsdorf (AT); Helmreich, Magdalena, 1110 Wien (AT); Fussenegger, Felix, 1040 Wien (AT)
(74) Vertreter: Seranski, Klaus

(56) Entgegenhaltungen:
- EP-A1- 3 117 868
- EP-A1- 3 320 926
- GB-A- 2 356 148
- US-A1- 2008 011 368

## Beschreibung

Die Erfindung betrifft eine Vorrichtung zum Verbinden von zwei jeweils ein Exsudatlumen und mindestens ein Belüftungslumen aufweisenden Leitungen für den medizinischen Bereich nach dem Oberbegriff des Patentanspruchs 1.

Bei der Unterdrucktherapie wird im Bereich einer zu behandelnden Wunde ein Unterdruck erzeugt, um so den Wundheilungsprozess zu fördern. Dazu wird die Wundregion über eine üblicherweise schlauchförmige Leitung an eine entsprechende Pumpe angeschlossen, mit der gleichzeitig Exsudat aus der Wundregion abgesaugt werden kann. Zusätzlich ist es regelmäßig erforderlich, den Druck in der Wundregion über ein gesondertes Leitungslumen zu messen, die Wundregion über gesonderte Leitungslumen zu belüften, Wundreinigungsmittel, Medikamente oder ähnliches zuzuführen, etc..

Bei einzelnen Indikationen kann es vorteilhaft sein, im Bereich der Wundregion eine kontinuierliche Strömung aufrechtzuerhalten. Bei anderen Indikationen kann es sinnvoll sein, einen konstanten Unterdruck aufrechtzuerhalten. Schließlich ist es bei einigen Indikationen auch sinnvoll, den Unterdruck in einer vorgegebenen zeitlichen Abfolge zu erzeugen und wieder zu reduzieren, d. h. einen intermittierenden Unterdruck im Bereich der Wundregion anzulegen. Dabei hat es sich als vorteilhaft erwiesen, wenn zur Belüftung zwei, drei oder mehr Belüftungslumen der mehrlumigen Leitung eingesetzt werden, während der Unterdruck über ein im Folgenden als Exsudatlumen bezeichnetes Leitungslumen mit größerem Leitungsquerschnitt erzeugt werden kann.

Zum Zweck der Unterdrucktherapie müssen die Leitungslumen mit einem geeigneten Unterdrucktherapiegerät verbunden werden. Dabei ist darauf zu achten, dass die Verbindung einerseits fest halten soll, sich andererseits bei Bedarf manuell kontrolliert, leicht, rasch und unkompliziert öffnen lässt.

Mit dem Unterdrucktherapiegerät wird einerseits der Unterdruck erzeugt und andererseits ggf. eine Belüftung bzw. Belüftung und/oder Druckmessung der Wundregion und/oder andere der vorstehend beschriebenen Aufgaben ausgeführt. Darüber hinaus ist es in vielen Fällen erforderlich, zwei, drei oder mehr mehrlumige Leitungen miteinander zu verbinden, wenn sich beispielsweise die Position des Unterdrucktherapiegeräts in Relation zum Patienten ändert und/oder die Länge der Leitung entsprechend angepasst werden muss. Bereits vorhandene Leitungen können dabei weiter benutzt werden, wenn Verlängerungsleitungen mit geeigneten Vorrichtungen dicht daran angeschlossen werden. Zusätzlich wird in vielen Fällen unter Einsatz von nur einem Unterdrucktherapiegerät eine Mehrzahl von Wunden versorgt, indem Belüftungslumen und Exsudatlumen der entsprechenden Leitungen über eine gemeinsame Verbindungsvorrichtung an ein gemeinsames Unterdrucktherapiegerät angeschlossen werden.

Im Stand der Technik ist es bekannt, mehrlumige Leitungen in Form von Schläuchen zu verwirklichen, die von mehreren Lumen in axialer Richtung durchsetzt sind. In diesem Fall ist es nicht mehr erforderlich, einzelne Schläuche miteinander zu verbinden. Vielmehr reicht es aus, wenn ein mehrlumiger Schlauch angeschlossen wird. Bei diesen bekannten mehrlumigen Schläuchen kann ein zentrales Exsudatlumen vorgesehen sein, welches koaxial zur Schlauchachse verläuft. Belüftungslumen des Schlauchs können radial versetzt angeordnet sein und den das Exsudatlumen begrenzenden Schlauchmantel in axialer Richtung parallel zur Schlauchachse durchsetzen.

Besonders vorteilhaft ist eine ungerade Anzahl von Belüftungslumen, besonders bevorzugt drei Belüftungslumen. Dabei können die radial versetzt angeordneten Belüftungslumen mit geringerem Querschnitt ausgestattet sein als das zentrale Exsudatlumen.

Verbindungsvorrichtungen der eingangs angesprochenen Art, mit denen der Anschluss und die Verbindung entsprechender mehrlumiger Schläuche erleichtert werden, sind in der EP 3 117 868 A1 angegeben. Der Offenbarungsgehalt dieser Schrift hinsichtlich der Konstruktion geeigneter Anschluss- bzw. Verbindungsvorrichtungen mit geeigneten Anschluss- bzw. Verbindungsstutzen, Dichtungshülsen, Kammerhülsen und Klemmhülsen wird hiermit durch ausdrückliche Inbezugnahme in diese Beschreibung aufgenommen.

Vorrichtungen nach dem Oberbegriff des Patentanspruchs 1 sind angegeben in der US 2008/011368 A1, der EP 3117868 A1 und der GB 2356148 A.

Insbesondere beim Einsatz der bekannten Verbindungsvorrichtungen zur Behandlung von zwei, drei oder mehr Wunden unter Verwendung von nur einem gemeinsamen Unterdrucktherapiegerät hat es sich gezeigt, dass es in einigen Fällen zu Therapieversagen kommt.

Angesichts dieser Probleme im Stand der Technik liegt der Erfindung die Aufgabe zugrunde, eine Weiterbildung der bekannten Verbindungs- bzw. Anschlussvorrichtungen bereitzustellen, mit denen ein Therapieversagen im Bereich einzelner Wunden zuverlässig ausgeschlossen werden kann.

Erfindungsgemäß wird diese Aufgabe durch die im kennzeichnenden Teil des Patentanspruchs 1 angegebene Weiterbildung der bekannten Vorrichtungen gelöst.

Die Erfindung geht auf die Erkenntnis zurück, dass es bei der Behandlung von zwei, drei oder mehr Wunden unter Einsatz von nur einem Unterdrucktherapiegerät zu einem Exsudatfluss zwischen den einzelnen Wunden über die Belüftungskanäle bzw. Belüftungslumen kommen kann. Das ist beispielsweise dann möglich, wenn es zu einem vollständigen Verschluss (Blockade) in einem Exsudatlumen und/oder einem Exsudatkanal kommt. In Abhängigkeit von den Unterdruckverhältnissen im Bereich der einzelnen Wunden kann es dann zu einem ungewollten Exsudatfluss über die Belüftungslumen des Systems von einer zur anderen Wunde kommen.

Zur Behandlung von zwei, drei oder mehr Wunden unter Einsatz von nur einem Unterdrucktherapiegerät ist die erfindungsgemäße Verbindungsvorrichtung vorzugweise so ausgelegt, dass jeder Wunde ein Belüftungskanal zugeordnet ist, sodass alle Wunden unter Einsatz von nur einer Verbindungsvorrichtung und nur einem Unterdrucktherapiegerät belüftet werden können. Im Rahmen der Erfindung hat es sich dabei als besonders vorteilhaft erwiesen, wenn die Barriereeinrichtung derart ausgelegt ist, dass bei Eintritt von Exsudat in einen Belüftungskanal, der zu einer Wunde führt, der Durchtritt des Exsudats bzw. anderer Flüssigkeiten in solche Belüftungskanalabschnitte verhindert wird, welche zu anderen Wunden führen.

Mit den erfindungsgemäß weitergebildeten Verbindungsvorrichtungen wird diesem Exsudatfluss mit Hilfe einer mindestens einem Belüftungskanal zugeordneten Barriereeinrichtung entgegengewirkt. Dadurch kann ein Austausch von Exsudatflüssigkeiten zwischen den einzelnen Wunden und ein darauf zurückzuführendes Therapieversagen verhindert werden.

Erfindungsgemäß weist die Barriereeinrichtung ein in dem Belüftungskanal angeordnetes, vorzugsweise gasdurchlässiges Barriereelement auf oder besteht daraus. Durch die Gasdurchlässigkeit wird die Funktionssicherheit der Verbindungsvorrichtung im normalen Betrieb gewährleistet. Die gewünschte Unterbindung des Durchtritts von Flüssigkeiten durch den Belüftungskanal kann sichergestellt werden, wenn das Barriereelement bei Kontakt mit Flüssigkeit aufquillt und so dem Durchtritt von Flüssigkeit durch den Belüftungskanal entgegenwirkt.

Zu diesem Zweck kann das Barriereelement einen Superabsorber aufweisen. Ein gleichzeitig gasdurchlässiges, andererseits aber quellfähiges Barriereelement kann bereitgestellt werden, indem ein offenporiger Schaum mit einem Superabsorberpulver versetzt wird, wobei das Superabsorberpulver bei Kontakt mit Flüssigkeit aufquillt und einen gelförmigen Zustand annimmt.

Im Rahmen der Erfindung hat es sich weiter als vorteilhaft erwiesen, wenn das Barriereelement genau in den Belüftungskanal eingepasst ist und diesen bei Kontakt mit Flüssigkeit durch Aufquellen verschließt. Im trockenen Zustand sind Superabsorber bzw. mit Superabsorbern versetzte offenporige Schäume luftdurchlässig und beeinflussen damit nicht die aktive Belüftung der Wunden. Insgesamt kann so die Funktion der Gesamtanordnung sichergestellt werden, ohne dass es zu einem durch einen unerwünschten Exsudatfluss durch das Belüftungslumen verursachten Therapieversagen kommt.

Der Exsudatkanal einer erfindungsgemäßen Verbindungsvorrichtung wird vorzugsweise zumindest teilweise von mindestens einem Verbindungsstutzen begrenzt, auf den ein die mehrlumige Leitung bildender mehrlumiger Schlauch aufschiebbar ist.

Bei der soeben beschriebenen Ausführungsform der Erfindung kann der Belüftungskanal radial außerhalb des Verbindungsstutzens gebildet sein, vorzugsweise zumindest abschnittweise von einer den Verbindungsstutzen zumindest teilweise umlaufenden Kammerhülse begrenzt werden, wobei das Barriereelement vorzugsweise zwischen Verbindungsstutzen und Kammerhülse angeordnet ist. Bei dieser Anordnung kann ein mehrlumiger Schlauch in den Ringspalt zwischen Verbindungsstutzen und Kammerhülse eingeführt werden. Die in dem Schlauchmantel gebildeten Belüftungslumen des mehrlumigen Schlauchs münden dann in diesen Ringspalt, welcher den Belüftungskanal bestimmt. Das Barriereelement kann angrenzend an eine Stirnfläche des mehrlumigen Schlauchs formschlüssig in den Ringspalt zwischen Verbindungsstutzen und Kammerhülse eingefügt sein und den von der Kammerhülse umlaufenen Verbindungsstutzen vorzugsweise vollständig umlaufen.

Die Verbindungsvorrichtung kann zwei, drei oder mehr fluidleitend miteinander verbundene Verbindungsstutzen aufweisen, von denen jeder von einer Kammerhülse umlaufen wird und jeder der Verbindungsstutzen kann über einen geeigneten mehrlumigen Schlauch mit einer Wundversorgungsanordnung verbunden werden, um so die gewünschte Vakuumtherapie im Bereich mehrerer Wunde zu gewährleisten.

Ähnlich wie bei Verbindungs- bzw. Anschlussvorrichtungen gemäß EP 3 117 868 A1 können die Verbindungsstutzen von einem gemeinsamen Grundkörper gebildet sein, welcher zur Bereitstellung einer Verteilerkammer für mindestens ein darin mündendes Belüftungslumen der angeschlossenen mehrlumigen Leitung ausgelegt ist, wobei vorzugsweise mindestens ein Belüftungskanal zum Ab- bzw. Einleiten eines Fluids aus der bzw. in die Verteilerkammer vorgesehen ist und die Verteilerkammer bei angeschlossener Leitung derart fluiddicht bezüglich mindestens eines Exsudatlumens abgedichtet ist, dass ein Fluidaustausch zwischen der Verteilerkammer und diesem Leitungslumen verhindert wird.

Die einzelnen auf die Verbindungsstutzen der verbindungsgemäßen Verbindungsvorrichtung aufgeschobenen mehrlumigen Schläuche können an ein gemeinsames Unterdrucktherapiegerät angeschlossen werden, wenn die Verbindungsvorrichtung einen zum Anschließen des Exsudatkanals an eine zum Erzeugen eines Unterdrucks ausgelegte Pumpe und/oder zum Anschließen einer Belüftungseinrichtung an den Belüftungskanal ausgelegten Anschlussbereich aufweist.

Dabei kann der Anschlussbereich einen vorzugsweise zumindest teilweise von einer Anschlusshülse umlaufenen und fluidleitend mit mindestens einem Verbindungsstutzen verbundenen Anschlussstutzen aufweisen, auf den eine mehrlumige Leitung aufschiebbar ist, die im Bereich ihres der Verbindungsvorrichtung abgewandten Endes mit dem Unterdrucktherapiegerät verbunden werden kann. Ähnlich wie der Verbindungsstutzen kann auch die Anschlusshülse zumindest teilweise von einer Anschlusshülse umlaufen sein, wobei der zur Verbindung mit dem Unterdrucktherapiegerät eingesetzte mehrlumige Schlauch in einen Ringspalt zwischen Anschlussstutzen und Anschlusshülse eingesetzt werden kann, wobei der Ringspalt fluidleitend mit dem Belüftungskanal verbunden sein kann, in den das Barriereelement eingesetzt ist.

Wenn die Verbindungsvorrichtung zwei Verbindungsstutzen aufweist, können die Verbindungsstutzen und der Anschlussstutzen etwa Y-förmig angeordnet sein. Dabei kann jedem der Verbindungsstutzen ein Barriereelement zugeordnet sein, welches den entsprechenden Verbindungsstutzen vorzugsweise vollständig umläuft.

Ein erfindungsgemäßes Behandlungskit weist eine erfindungsgemäß mit einer Barriereeinrichtung ausgestattete Verbindungsvorrichtung und eine auf einen Verbindungs- oder Anschlussstutzen davon aufschiebbare mehrlumige Leitung auf.

Vorzugsweise weist die mehrlumige Leitung einen Schlauch mit einem von einem Schlauchmantel begrenzten zentralen Exsudatlumen und mindestens einem den Schlauchmantel in einer parallel zur Schlauchachse und parallel zum Exsudatlumen verlaufenden Richtung durchsetzenden Belüftungslumen auf, wobei das mindestens eine Belüftungslumen nach Anschluss an die Verbindungsvorrichtung in den Belüftungskanal mündet und/oder der Verbindungs- und/oder Anschlussstutzen in das Exsudatlumen einführbar ist.

Nachstehend wird die Erfindung, auf die hinsichtlich aller erfindungswesentlichen und in der Beschreibung nicht ausdrücklich hervorgehobenen Einzelheiten Bezug genommen wird, erläutert.

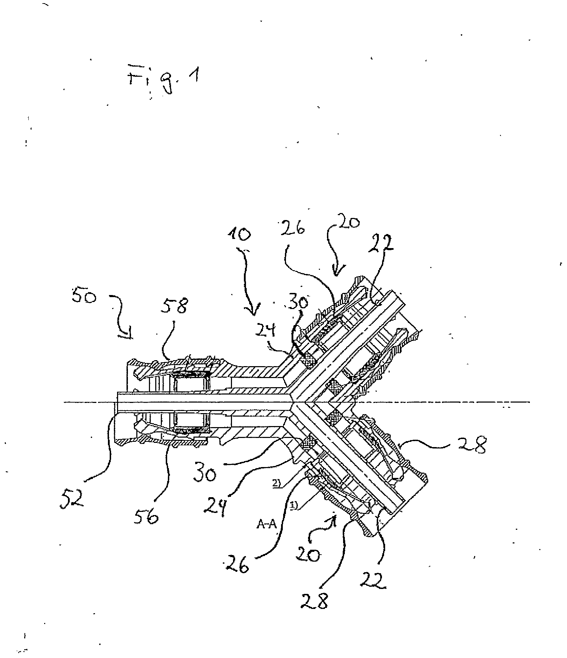

Die einzige Figur der Zeichnung zeigt eine Schnittdarstellung einer erfindungsgemäßen Verbindungsvorrichtung. Die in der Zeichnung dargestellte Verbindungsvorrichtung 10 umfasst zwei Verbindungsbereiche 20 und einen Anschlussbereich 50. Jeder der Verbindungsbereiche 20 weist einen von einem Exsudatkanal durchsetzten Verbindungsstutzen 22 auf, wobei die Exsudatkanäle in einen gemeinsamen Anschlussstutzen 52 des Anschlussbereichs 50 münden. Die Verbindungsstutzen 22 werden jeweils von einer Verbindungshülse 24 umlaufen, wobei zwischen Verbindungsstutzen 22 und Verbindungshülse 24 ein Ringspalt gebildet ist.

In den einzelnen Ringspalten ist jeweils eine Dichthülse 26 angeordnet. Zwischen Verbindungsstutzen 22 und Dichthülse 26 sind Ringspalte gebildet, in die die Schlauchmäntel mehrlumiger Schläuche eingeführt werden können, wobei die Verbindungsstutzen 22 in ein zentrales Exsudatlumen der mehrlumigen Schläuche eingeführt sind.

Die Schlauchmäntel der Verbindungsschläuche können von Belüftungslumen durchsetzt werden, welche in den Ringspalt münden. Die Kammerhülsen 24 werden von einer Klemmhülse 28 umlaufen, welche zum Arretieren der auf die Verbindungsstutzen 22 aufgeschobenen Schläuche in den zwischen den Kammerhülsen 24 und den Verbindungsstutzen 22 gebildeten Ringspalten dient.

Der Anschlussbereich 50 ist ähnlich ausgeführt. Er weist ebenfalls einen Anschlussstutzen 52, eine Anschlusshülse 54, eine Dichthülse 56 und eine Klemmhülse 58 auf. Die bislang beschriebenen Bestandteile einer erfindungsgemäßen Verbindungsvorrichtung entsprechen in Aufbau und Funktion der in der EP 3 117 868 A1 beschriebenen Anschlussvorrichtung. Hinsichtlich Aufbau und Funktion von Anschlussstutzen, Verbindungsstutzen, Anschlusshülse, Verbindungshülse, Dichthülse und Klemmhülse wird Bezug genommen auf den Offenbarungsgehalt dieser Schrift.

In dem Ringspalt zwischen Verbindungsstutzen 22 und Verbindungshülse 24 ist auf der dem Mündungsbereich abgewandten Seite der Dichtungshülse 26 ein Barriereelement 30 vorgesehen. Das Barriereelement 30 ist insgesamt ringförmig und gasdurchlässig ausgeführt. Bei der anhand der Zeichnung dargestellten Ausführungsform der Erfindung ist das Barriereelement 30 mit einem Superabsorber versetzt, der bei Kontakt mit Flüssigkeit aufquillt.

Wenn in Abhängigkeit von den Unterdruckverhältnissen bei einer Blockade eines Exsudatlumens eines mit einem Verbindungsbereich 20 verbundenen mehrlumigen Schlauch Exsudatflüssigkeit in den zwischen Verbindungsstutzen 22 und Verbindungshülse 24 begrenzten Belüftungskanal eindringt, kommt es zu einem Kontakt zwischen Exsudatflüssigkeit und Barriereelement 30. Das führt zu einem Aufquellen des Barriereelements 30. Dadurch wird der Exsudatfluss durch den zwischen Verbindungsstutzen 22 und Verbindungshülse 24 begrenzten Belüftungskanal verhindert.

Die Erfindung ist nicht auf das anhand der Zeichnung erläuterte Ausführungsbeispiel beschränkt. Vielmehr ist auch an den Einsatz von Verbindungsvorrichtungen mit drei, vier oder mehr Verbindungsbereichen gedacht. Es können auch zwei, drei oder mehr Anschlussbereiche vorgesehene sein. Es ist auch an eine andere als die dargestellte Y-förmige Anordnung von Verbindungs- und Anschlussstutzen gedacht. Wesentlich ist, dass in einem Belüftungskanal der Vorrichtung ein Barriereelement angeordnet ist, das bei Kontakt mit Flüssigkeit aufquillt und einen Exsudatfluss durch den Belüftungskanal verhindert.

## Patentansprüche

1. Vorrichtung zum Verbinden von zwei, jeweils mindestens ein Exsudatlumen und mindestens ein Belüftungslumen aufweisenden Leitungen für den medizinischen Bereich, geeignet für die Wundversorgung unter Einsatz der Unterdrucktherapie, mit mindestens zwei fluidleitenden Kanälen, von denen mindestens ein Exsudatkanal zum fluidleitenden Verbinden der Exsudatlumen der mehrlumigen Leitungen und mindestens ein Belüftungskanal zum fluidleitenden Verbinden der Belüftungslumen der mehrlumigen Leitungen ausgelegt ist, wobei mindestens einem Belüftungskanal eine dem Durchtritt von Flüssigkeiten entgegenwirkende Barriereeinrichtung (30) zugeordnet ist , **dadurch gekennzeichnet, dass** die Barriereeinrichtung ein in dem Belüftungskanal angeordnetes gasdurchlässiges Barriereelement (30) aufweist oder ist.

2. Vorrichtung nach Anspruch 1, **dadurch gekennzeichnet, dass** das Barriereelement (30) bei Kontakt mit Flüssigkeit aufquillt und so dem Durchtritt von Flüssigkeit durch den Belüftungskanal entgegenwirkt.

3. Vorrichtung nach Anspruch 2, **dadurch gekennzeichnet, dass** das Barriereelement einen Superabsorber aufweist.

4. Vorrichtung nach einem der vorhergehenden Ansprüche, **dadurch gekennzeichnet, dass** der Exsudatkanal zumindest teilweise von mindestens einem Verbindungsstutzen (22) begrenzt wird, auf den ein die mehrlumige Leitung bildender mehrlumiger Schlauch aufschiebbar ist.

5. Vorrichtung nach Anspruch 4, **dadurch gekennzeichnet, dass** der Belüftungskanal radial außerhalb des Verbindungsstutzens gebildet ist, vorzugsweise zumindest abschnittweise von einer den Verbindungsstutzen (22) zumindest teilweise umlaufenden Kammerhülse (24) begrenzt wird, wobei das Barriereelement (30) vorzugsweise zwischen Verbindungsstutzen (22) und Kammerhülse (24) angeordnet ist.

6. Vorrichtung nach einem der vorhergehenden Ansprüche, **gekennzeichnet durch** einen das Anschließen des Exsudatkanals an eine zum Erzeugen eines Unterdrucks ausgelegte Pumpe und/oder zum Anschließen einer Belüftungseinrichtung an den Belüftungskanal ausgelegten Anschlussbereich (50).

7. Vorrichtung nach Anspruch 6, **dadurch gekennzeichnet, dass** der Anschlussbereich (50) einen vorzugsweise zumindest teilweise von einer Anschlusshülse (54) umlaufenen und fluidleitend mit dem mindestens einen Verbindungsstutzen (22) verbundenen Anschlussstutzen (52) aufweist.

8. Vorrichtung nach Anspruch 7 mit zwei Verbindungsstutzen (22), wobei die Verbindungsstutzen (22) und der Anschlussstutzen (52) etwa Y-förmig angeordnet sind.

## Claims

1. Device for connecting two lines adapted for the medical field, each having at least one exudate lumen and at least one ventilation lumen, and appropriate for wound care using negative pressure therapy, wherein the device comprises at least two fluid-conducting channels, of which at least one exudate channel is configured to be connected, in a fluid-conducting manner, to the exudate lumens of the multi-lumen lines and at least one ventilation channel is configured to be connected, in a fluid-conducting manner, to the ventilation lumens of the multi-lumen lines, wherein a barrier device (30) counteracting the passage of liquids is associated with at least one ventilation channel, **characterised in that** the barrier device includes or is a gas-permeable barrier element (30) arranged in the ventilation channel.

2. Device according to claim 1, **characterised in that** the barrier element (30) swells on contact with liquid and thus counteracts the passage of liquid through the ventilation channel.

3. Device according to claim 2, **characterised in that** the barrier element includes a super absorber.

4. Device according to one of the preceding claims, **characterised in that** the exudate channel is at least partially delimited by at least one connecting piece (22) onto which a multi-lumen tube forming the multi-lumen line can be pushed.

5. Device according to claim 4, **characterised in that** the ventilation channel is formed radially outside of the connecting piece, and is delimited preferably at least in sections by a chamber sleeve (24) which at least partially surrounds the connecting piece (22), wherein the barrier element (30) is preferably arranged between the connecting piece (22) and the chamber sleeve (24).

6. Device according to one of the preceding claims, **characterised by** a connection area (50) configured for the connection of the exudate channel to a pump configured to generate a negative pressure and/or for the connection of a ventilation device to the ventilation channel.

7. Device according to claim 6, **characterised in that** the connection area (50) has a connection piece (52) which is preferably at least partially surrounded by a connection sleeve (54) and which is connected in a fluid-conducting manner to the at least one connecting piece (22).

8. Device according to claim 7, comprising two connecting pieces (22), wherein said connecting pieces (22) and said connection piece (52) are arranged approximately in a Y-shape.

## Revendications

1. Dispositif destiné à raccorder deux conduites destinées au domaine médical, présentant chacune au moins une lumière pour exsudat et au moins une lumière d'aération et adaptées au soin des plaies par pression négative, le dispositif comportant au moins deux canaux d'évacuation de fluide, dont au moins un canal pour exsudat conçu pour être raccordé, de façon permettant un écoulement de fluide, aux lumières pour exsudat des conduites à plusieurs lumières et au moins un canal d'aération conçu pour être raccordé, de façon permettant un écoulement de fluide, aux lumières d'aération des conduites à plusieurs lumières, un dispositif barrière (30) qui empêche la pénétration de liquides étant associé à au moins un canal d'aération, **caractérisé en ce que** le dispositif barrière présente ou consiste en un élément barrière (30) perméable aux gaz, disposé dans le canal d'aération.

2. Dispositif selon la revendication 1, **caractérisé en ce que** l'élément barrière (30) gonfle au contact d'un liquide et empêche ainsi la pénétration de liquide dans le canal d'aération.

3. Dispositif selon la revendication 2, **caractérisé en ce que** l'élément barrière présente un superabsorbeur.

4. Dispositif selon l'une quelconque des revendications précédentes, **caractérisé en ce que** le canal pour exsudat est délimité au moins partiellement par au moins une tubulure de raccordement (22) sur laquelle peut être emboîté un tuyau à plusieurs lumières formant la conduite à plusieurs lumières.

5. Dispositif selon la revendication 4, **caractérisé en ce que** le canal d'aération est formé radialement sur l'extérieur de la tubulure de raccordement, en étant délimité de préférence au moins par sections par un manchon formant chambre (24) entourant au moins partiellement la tubulure de raccordement (22), ledit élément barrière (30) étant disposé de préférence entre la tubulure de raccordement (22) et le manchon formant chambre (24).

6. Dispositif selon l'une quelconque des revendications précédentes, **caractérisé par** une section de branchement (50) conçue pour le branchement du canal pour exsudat à une pompe conçue pour produire une pression négative et/ou pour le branchement d'un dispositif d'aération au canal d'aération.

7. Dispositif selon la revendication 6, **caractérisé en ce que** la section de branchement (50) présente une tubulure de branchement (52) entourée de préférence au moins partiellement par un manchon de branchement (54) et raccordée de façon permettant un écoulement de fluide à l'au moins une tubulure de raccordement (22).

8. Dispositif selon la revendication 7, comportant deux tubulures de raccordement (22), lesdites tubulures de raccordement (22) et ladite tubulure de branchement (52) étant agencées approximativement en Y.
